# FASCICULE DE BREVET EUROPEEN

(11) **EP 1 240 874 B1**
(45) Date de publication et mention de la délivrance du brevet: **09.12.2009**
(21) Numéro de dépôt: 02290630.9
(22) Date de dépôt: 13.03.2002
(51) Int. Cl.: A61B 17/70

(54) **Organe d'ancrage avec cale**
Verankerungselement mit Keil
Anchor member with wedge

(30) Priorité: 15.03.2001 FR 0103514
(43) Date de publication de la demande: 18.09.2002
(73) Titulaire: Stryker Spine, 33610 Cestas (FR)
(72) Inventeur: Saint Martin, Pierre Henri, 33700 Merignac (FR)
(74) Mandataire: Le Forestier, Eric

(56) Documents cités:
- EP-A- 0 997 107
- FR-A- 2 555 645
- FR-A- 2 659 546
- US-A- 5 738 685
- US-A- 5 976 141

## Description

L' invention concerne les systèmes d'ostéosynthèse rachidiens.

Les systèmes d'ostéosynthèse rachidiens comportent généralement des vis osseuses, comme des vis pédiculaires. Il arrive que, durant l'intervention chirurgicale, une ou plusieurs vis ne soit pas mise à fond, c'est-à-dire que le filet cortical ne soit pas intégralement engagé dans l'os de la vertèbre. Cela se produit pour différentes raisons : ce peut être du fait que la vis est trop longue et que, si elle était mise à fond, l'extrémité distale du filet réémergerait de l'os sur la face opposée à la face de pénétration de la vis, portant atteinte aux tissus se situant de ce côté. Une autre raison est un défaut anatomique conduisant au fait que, si la vis était mise à fond, elle serait plus enfoncée que les autres vis du système d'ostéosynthèse utilisé, ce qui conduirait à un cintrage exagéré et difficile à réaliser de l'organe de liaison. Pour éviter ceci, le chirurgien ne visse pas complètement la vis dans l'os, laissant la partie proximale du filet cortical désengagée de l'os. Dans tous les cas, cela a pour inconvénient de rendre instable la vis dans l'os car celle-ci n'est pas serrée et de laisser des filets osseux hors de l'os dans lequel l'organe est ancré, ce qui conduit à blesser les tissus environnants du fait de la présence des angles saillants du filetage.

EP 0 997107 présente un organe d'ancrage pour fixer l'extrémité d'une vis pédiculaire.

Un but de l'invention est de fournir un système permettant de mieux stabiliser un organe d'ancrage tout en isolant davantage la partie d'ancrage non engagée de l'environnement adjacent.

Pour cela, on prévoit, selon l'invention, un ensemble d'ostéosynthèse rachidien comme décrit dans la revendication 1.

Ainsi, la partie des moyens d'ancrage de l'organe d'ancrage qui n'est pas engagée dans l'os se retrouve au moins partiellement entourée par la cale qui l'isole de l'environnement adjacent. D'autre part, si la cale vient en appui sur l'os, elle assure une meilleure stabilité. Par conséquent, l'organe d'ancrage est stabilisé et la partie non engagée des moyens d'ancrage ne peut plus provoquer de blessure aux tissus environnant l'organe d'ancrage.

Avantageusement, la cale présentant une épaisseur e, elle comporte une fente sur toute l'épaisseur e.

Avantageusement, la fente est agencée de sorte que la cale forme un anneau non fermé.

Avantageusement, la fente a une largeur sensiblement égale à une dimension des moyens d'ancrage.
Ainsi, la cale peut être mise en place sur l'organe d'ancrage alors que ce dernier est déjà ancré à l'os.

Avantageusement, les moyens de retenue comportent au moins une lèvre de l'organe d'ancrage et au moins une lèvre de la cale complémentaire de la lèvre de l'organe d'ancrage pour venir en prise avec celle-ci.
Ainsi, la cale est clipsée à l'organe d'ancrage, lors d'un mouvement simple.

Avantageusement, la cale comporte au moins une rainure au niveau de la lèvre.

Avantageusement, la cale étant une première cale et les moyens de retenue étant des premiers moyens de retenue, il comprend une seconde cale comportant un orifice traversant apte à recevoir les moyens d'ancrage, l'ensemble comportant des seconds moyens de retenue pour la retenue de la seconde cale contre la première cale.

Avantageusement, les seconds moyens de retenue sont identiques aux premiers moyens de retenue.

Avantageusement, la seconde cale est identique à la première cale.

On prévoit un système d'ostéosynthèse comportant au moins un ensemble présentant au moins une des caractéristiques précédentes.

On prévoit aussi une méthode chirurgicale présentant des étapes de mise en place de la cale sur l'organe d'ancrage, puis d'insertion de l'organe d'ancrage dans l'os.

Avantageusement, la méthode présente des étapes de mise en place de l'organe d'ancrage dans l'os, puis de mise en place de la cale sur l'organe d'ancrage.

Avantageusement, la méthode présente une étape supplémentaire d'ancrage de l'organe d'ancrage jusqu'à ce que la cale vienne en contact avec l'os.

D'autres caractéristiques et avantages de l'invention apparaîtront à la lecture de la description ci-après d'un mode de réalisation préféré ainsi que de variantes. Aux dessins annexés :
- la figure 1 est une vue en trois dimensions d'un premier mode de réalisation de l'invention, en montage ;
- la figure 2 est une vue en trois dimensions de la cale du premier mode de réalisation ;
- la figure 3 est une vue en coupe selon III-III du second mode de réalisation de l'invention de la figure 1 ;
- la figure 4 est vue de la section selon IV-IV de la cale du premier mode de réalisation de la figure 2 ;
- la figure 5 est une vue en trois dimensions d'un second mode de réalisation de l'invention, avant montage ;
- la figure 6 est une vue en trois dimensions du second mode de réalisation de la figure 5, en montage ;
- la figure 7 est une vue en trois dimensions de la cale du second mode de réalisation ;
- la figure 8 est une vue en trois dimensions d'une cale d'un troisième mode de réalisation de l'invention ; et,
- la figure 9 est une vue en trois dimensions d'un quatrième mode de réalisation de la cale de l'invention.

Pour tous les modes de réalisation qui vont être décrits, seul un organe d'ancrage 2 est représenté, le reste du système d'ostéosynthèse ne l'étant pas.

De manière commune à tous les modes de réalisation, un système d'ostéosynthèse comprend une pluralité d'organes d'ancrage 2, ici des vis osseuses monoaxiales, au moins un élément de liaison (non représenté), ici des tiges de liaison de section circulaire, ainsi que des moyens de verrouillage (non représentés) des éléments de liaison sur les organes d'ancrage, ici des verrous. Chaque organe d'ancrage 2 du système d'ostéosynthèse est constitué de deux parties principales : une partie inférieure 21 ici de forme de cylindrique à section circulaire présentant un filetage osseux, cette partie étant apte à venir s'engager et à s'ancrer dans l'os. Une partie supérieure 22 de l'organe d'ancrage, appelée « tête de vis », comprend deux branches parallèles s'étendant en regard l'une de l'autre l'une de l'autre et délimitant une ouverture en « U » apte à recevoir l'élément de liaison. Les faces intérieures en vis-à-vis des branches formant le « U » présentent un filetage apte à coopérer avec un filetage complémentaire des moyens de verrouillage. De tels organes d'ancrage sont décrits dans les brevets FR 2 642 643 et FR 2 659 546 auxquels on se reportera pour plus de détails. La partie supérieure 22 et la partie inférieure 21 de chaque organe d'ancrage 2 sont liées l'une à l'autre en formant une surface 24 qui présente une entaille circulaire 23 de section sensiblement en « V » et s'étendant sur toute la circonférence de la surface 24.

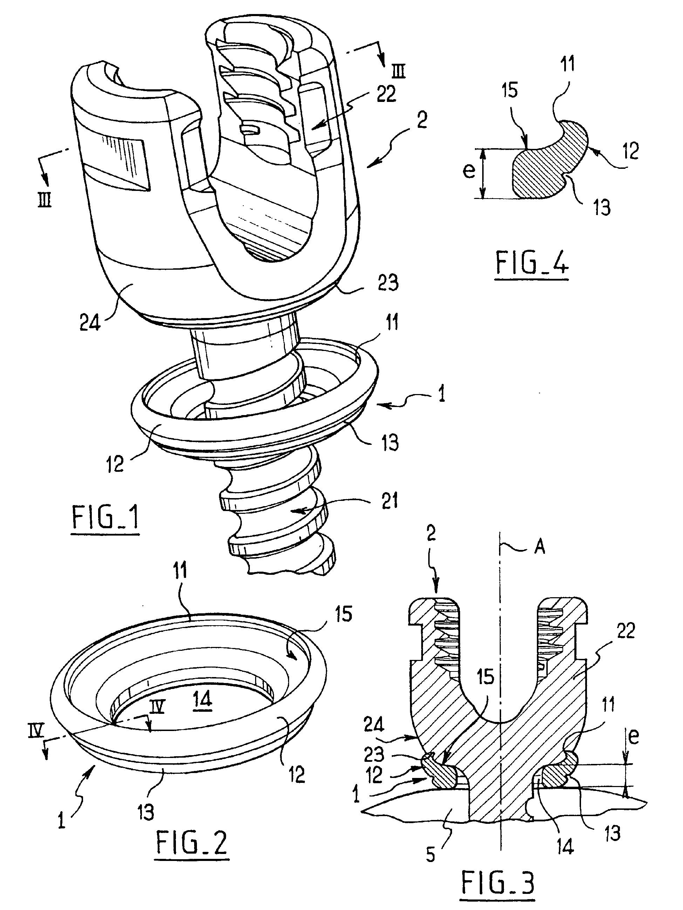

En référence aux figures 1 à 4, nous allons décrire un premier mode de réalisation de l'invention. La cale 1 a une forme annulaire et présente une surface externe latérale 12 et une surface interne latérale 15, la surface interne latérale 15 délimitant une ouverture centrale traversante 14. L'extrémité supérieure de la surface interne 15 comporte une lèvre 11 sur toute sa circonférence. La surface latérale externe 12 comporte dans sa partie inférieure une entaille circulaire 13 de section sensiblement en V et s'étendant sur toute la circonférence de l'anneau. La surface latérale interne 15 est sensiblement complémentaire de la partie de la face 24 de l'organe d'ancrage 2 situé entre l'entaille 23 et la partie inférieure 21 de l'organe d'ancrage 2. D'autre part, la partie de la surface latérale externe 12 située en dessous de l'entaille circulaire 13 de la cale 1 est sensiblement complémentaire avec la partie supérieure de la surface latérale interne 15. La cale 1 présente une épaisseur utile e mesurée parallèlement à un axe A perpendiculaire au plan générale de la cale.

On va maintenant décrire l'utilisation du premier mode de réalisation de l'invention. Le chirurgien, avant d'engager son organe d'ancrage dans l'os à l'emplacement désiré, monte ensemble l'organe d'ancrage 2 avec la cale 1. Pour cela, il enfile la partie 21 présentant le filetage osseux à travers l'orifice 14 de la cale 1 jusqu'à ce que la lèvre 11 vienne en contact avec la surface 24 de l'organe d'ancrage. A ce moment, la lèvre 11 se situe à proximité de l'entaille 23. Le chirurgien applique un effort supplémentaire sur la cale pour que la lèvre 11 vienne s'engager dans l'entaille 23. Pour obtenir ce résultat, le chirurgien a joué sur l'élasticité du matériau constituant la cale 1. Ce matériau est biocompatible pour pouvoir être toléré par l'organisme humain. Ce peut être de l'acier inoxydable, comme du 316L, du titane ou bien un alliage de titane, comme le TA6V4, ou bien un polymère comme du PEEK (polyéther éther kétone). Une fois cet assemblage réalisé, le chirurgien met en place dans le patient l'organe d'ancrage de manière à ce que, de préférence, la cale vienne en contact avec l'os 5, comme illustrée en figure 3. Puis, il poursuit son opération chirurgicale.

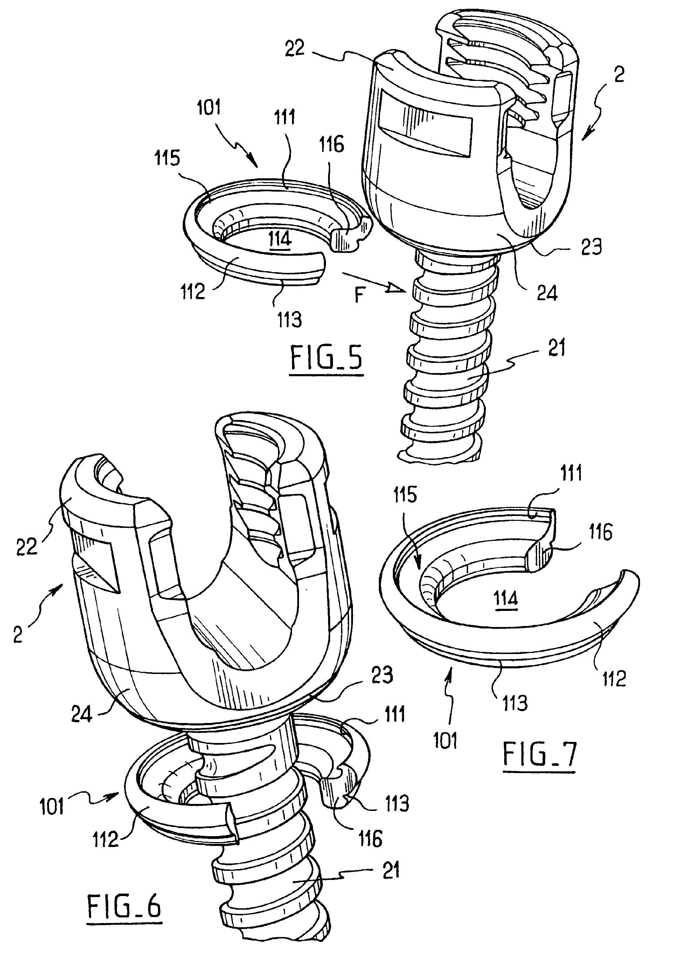

En référence aux figures 5 à 7, nous allons décrire un second mode de réalisation de l'invention. la cale 101 est très similaire à la cale 1 du mode de réalisation précédent : cette cale est formée d'un anneau de section identique à la section de l'anneau formant la cale 1. La surface 115 est identique à la surface 15 ; de même, la surface 112 est identique à la surface 12. L'extrémité supérieure de la surface 115 présente une lèvre 111 identique à la lèvre 11. De même, la surface 112 présente une entaille 113 identique à l'entaille 13. La surface interne latérale 115 délimite un orifice 114. La différence se situe dans le fait que la cale 101 présente une fente 116, formant ainsi un anneau non fermé. Cette fente 116 présente une largeur d'ouverture sensiblement équivalente au diamètre de la partie inférieure 21 de l'organe d'ancrage 2.

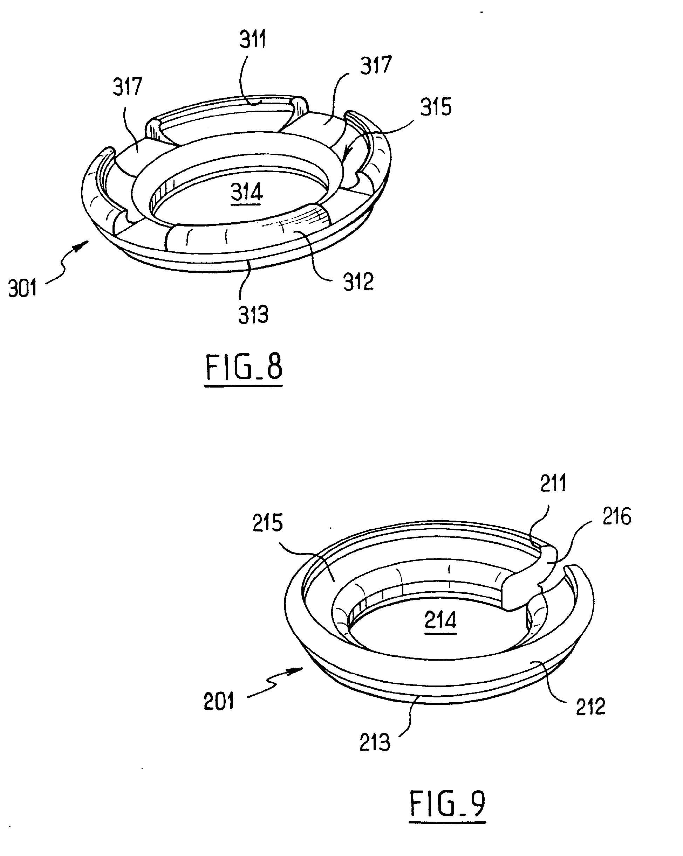

En utilisation, le chirurgien peut monter la cale 101 sur l'organe d'ancrage 2 avant de mettre en place dans le patient l'organe d'ancrage 2, ou bien il peut installer la cale 101 après avoir positionné dans le patient l'organe d'ancrage 2. Dans le premier cas, le montage s'effectue de la même manière que lors du montage du mode précédent de réalisation de l'invention. Dans le deuxième cas, le chirurgien a mis en place l'organe d'ancrage 2 et une partie d'ancrage de la partie 21 de l'organe d'ancrage 2 n'est pas engagée dans l'os et fait saillie à l'extérieur de celui-ci. Pour protéger les tissus et les organes biologiques environnants, d'une part et, d'autre part, pour stabiliser au mieux l'organe d'ancrage, le chirurgien va insérer une cale entre la tête 22 de l'organe d'ancrage 2 et la surface de l'os. Pour cela, il clipse la cale 101 sous la tête 22 de l'organe d'ancrage 2 comme suit. Comme il est indiqué sur la figure 5 par la flèche F, la partie 21 est insérée dans l'orifice 114 à travers la fente 116. Au moment de passer la fente 116, celle-ci s'écarte légèrement du fait de l'élasticité du matériau de la cale pour laisser passer la partie 21. Une fois celle-ci dans l'orifice 114, la fente 116 reprend son ouverture précédente en se refermant par élasticité du matériau formant la cale 101. Le chirurgien n'a alors plus qu'à exercer un effort similaire à l'effort qu'il exercerait pour le mode de réalisation précédent pour venir insérer la lèvre 111 de la cale 101 dans l'entaille 23 de l'organe d'ancrage 2. La cale 101 est montée sur l'organe d'ancrage 2 tel qu'illustré à la figure 8. Ensuite, le chirurgien poursuit normalement son opération chirurgicale.

En référence à la figure 8, on va décrire un troisième mode de réalisation de l'invention. La cale 301 se différentie de la cale 1 par le fait que des rainures 317 uniformément réparties sur toute la circonférence d'une partie supérieure de la cale 301 partagent en secteurs angulaires sensiblement identiques la lèvre 311. Ici, les rainures 317 sont au nombre de quatre. Ainsi, lors du montage, la déformation élastique de la cale 301 est facilitée lors de l'insertion de la lèvre 311 dans l'entaille 23 de l'organe d'ancrage 2. Par conséquent, le chirurgien fournit un effort plus faible que celui qu'il fournit pour la cale 1 du premier mode de réalisation pour clipser la cale 301 sur l'organe d'ancrage 2.
L'utilisation de la cale 301 est identique à l'utilisation de la cale 1 du premier mode de réalisation.

En référence à la figure 9, selon un quatrième mode de réalisation de l'invention, la cale 201 est identique à la cale 101 si ce n'est que l'ouverture de la fente 216 est beaucoup plus petite. Cette fente 216 ne permet pas d'introduire latéralement dans l'orifice 214 la partie 21 de l'organe d'ancrage 2. Le montage de cette cale 201 est identique au montage de la cale 1 précédemment décrit. Tout comme pour le troisième mode de réalisation de l'invention, la déformation de la bague 201 est facilitée par la présence de la fente 216 lors du clipsage pour introduire la lèvre 211 dans l'entaille 23. Par conséquent, le chirurgien fournit un effort plus faible que celui qu'il fournit pour la cale 1 du premier mode de réalisation pour monter la cale 201 sur l'organe d'ancrage 2.

Pour tous les modes de réalisation, les cales comportant sur leur surface extérieure une entaille identique à l'entaille réalisée sur l'organe d'ancrage, celles-ci peuvent alors être empilées au gré du chirurgien, la lèvre de la cale inférieure venant se clipser dans l'entaille de la cale supérieure. On peut ainsi enfiler et monter deux cales ou plus sur l'organe d'ancrage.

Bien entendu, il est possible d'apporter à la présente invention de nombreuses modifications sans pour autant sortir du cadre de celle-ci.
Par exemple, les lèvres ainsi que l'entaille pourront être remplacées par des surfaces coniques complémentaires, délimitant des cônes dits « Morse ».
Les lèvres pourront être remplacées par un filet alors que l'entaille sera remplacée par une gorge surmontant un filetage complémentaire du filet. Ainsi la cale sera fixée sur le filetage jusqu'à ce que le filet vienne dans la gorge où il sera retenu prisonnier.
Les lèvres pourront être remplacées par une pluralité d'ergots faisant saillie vers l'orifice central de la cale et uniformément répartis sur la circonférence de la cale, alors que l'entaille sera remplacée par des logements en nombre égal au nombre d'ergots, logements aptes à recevoir et à retenir lesdits ergots.
L'organe d'ancrage pourra être toute sorte de vis osseuse de type polyaxiale ou monoaxiale. La liaison entre l'organe d'ancrage et la cale pourra se situer au niveau de la tête ou bien sur la partie d'ancrage de l'organe.

## Revendications

1. Ensemble d'ostéosynthèse rachidien comportant :
- un organe d'ancrage osseux (2) comportant une tête (22) et des moyens d'ancrage osseux (21), et
- au moins une cale (1;101;201;301) comportant un orifice traversant (14;114;214;314) apte à recevoir l'organe d'ancrage (21), ladite cale étant empilable de sorte que l'on peut enfiler et monter deux cales ou plus sur l'organe d'ancrage,
dans lequel la cale empilable est positionnée entre la tête de l'organe d'ancrage osseux et les moyens d'ancrage osseux, et la cale empilable est apte à être montée sur la tête de l'organe d'ancrage osseux,
dans lequel l'organe d'ancrage comprend une entaille (23) et la cale empilable comporte, en outre, une lèvre (11;111;211;311) complémentaire de l'entaille (23), de sorte qu'un engagement de la lèvre avec l'entaille permette un montage de la cale empilable sur l'organe d'ancrage osseux.

2. Ensemble selon la revendication 1, **caractérisé en ce qu'**une fente (116;216) est agencée de sorte que la cale empilable forme un anneau non fermé.

3. Ensemble selon la revendication 2, **caractérisé en ce que** la cale empilable est déformable élastiquement afin de permettre l'insertion des moyens d'ancrage osseux à travers la fente.

4. Ensemble selon l'une des revendications 1 à 3, **caractérisé en ce que** la lèvre de la cale empilable est discontinue.

5. Ensemble selon l'une des revendications 1 à 4, **caractérisé en ce que** la cale empilable comporte une entaille (13;113;213;313) complémentaire de la lèvre (11;111;211;311).

6. Ensemble selon la revendication 5, **caractérisé en ce que** la cale empilable est apte à être montée sur une autre cale empilable par engagement de, respectivement, la lèvre dans l'entaille.

7. Ensemble selon les revendications 5 ou 6, **caractérisé en ce qu'**au moins deux cales empilable sont montées ensemble.

8. Ensemble selon la revendication 7, **caractérisé en ce qu'**une première cale empilable comprenant des premières lèvres et entailles, et une deuxième cale empilable comprenant des deuxièmes lèvres et entailles, la première cale empilable est montée sur l'organe d'ancrage par engagement de la première lèvre dans l'entaille de l'organe d'ancrage, et la deuxième cale empilable est montée sur la première cale empilable par engagement de la deuxième lèvre dans la première entaille.

9. Ensemble selon la revendication 8, **caractérisé en ce que** la deuxième cale empilable est positionnée entre la première cale empilable et les moyens d'ancrage osseux.

10. Ensemble selon la revendication 1, **caractérisé en ce qu'**une pluralité de cales empilable est positionnée entre la tête et les moyens d'ancrage osseux.

11. Ensemble selon l'une des revendications 1 à 10, **caractérisé en ce que** la cale empilable est apte à être montée sur l'organe d'ancrage pour un engagement par déformation élastique de la lèvre de la cale empilable sur l'entaille de l'organe d'ancrage.

## Claims

1. Spinal osteosynthesis assembly comprising:
- a bone anchoring member (2) comprising a head (22) and bone anchoring means (21), and
- at least one packer (1; 101; 201; 301) comprising a through-orifice (14; 114; 214; 314) capable of receiving the anchoring member (21), the said packer being stackable so that two or more packers can be stacked and mounted on the anchoring member, in which assembly the stackable packer is positioned between the head of the bone anchoring member and the bone anchoring means, and the stackable packer can be mounted on the head of the bone anchoring member, in which assembly the anchoring member comprises a cut (23) and the stackable packer additionally comprises a lip (11; 111; 211; 311) that complements the cut (23), such that engaging the lip with the cut allows the stackable packer to be mounted on the bone anchoring member.

2. Assembly according to Claim 1, **characterized in that** a slot (116; 216) is arranged in such a way that the stackable packer forms a non-closed annulus.

3. Assembly according to Claim 2, **characterized in that** the stackable packer is elastically deformable so as to allow the bone anchoring means to be inserted through the slot.

4. Assembly according to one of Claims 1 to 3, **characterized in that** the lip of the stackable packer is discontinuous.

5. Assembly according to one of Claims 1 to 4, **characterized in that** the stackable packer comprises a cut (13; 113; 213; 313) that complements the lip (11; 111; 211; 311).

6. Assembly according to Claim 5, **characterized in that** the stackable packer can be mounted on another stackable packer by, respectively, engaging the lip in the cut.

7. Assembly according to Claims 5 or 6, **characterized in that** at least two stackable packers are mounted together.

8. Assembly according to Claim 7, **characterized in that**, with a first stackable packer comprising first lips and cuts and with a second stackable packer comprising second lips and cuts, the first stackable packer is mounted on the anchoring member by engaging the first lip in the cut of the anchoring member, and the second stackable packer is mounted on the first stackable packer by engaging the second lip in the first cut.

9. Assembly according to Claim 8, **characterized in that** the second stackable packer is positioned between the first stackable packer and the bone anchoring means.

10. Assembly according to Claim 1, **characterized in that** a plurality of stackable packers is positioned between the head and the bone anchoring means.

11. Assembly according to one of Claims 1 to 10, **characterized in that** the stackable packer can be mounted on the anchoring member by engaging the lip of the stackable packer on the cut of the anchoring member by elastic deformation.

## Patentansprüche

1. Anordnung zur Wirbelosteosynthese, welches umfasst:
- ein Knochenverankerungselement (2), das einen Kopf (22) und Knochenverankerungsmittel (21) umfasst, und
- wenigstens einen Keil (1;101;201;301), der eine Durchgangsöffnung (14;114;214;314) umfasst, die ausgelegt ist, um das Verankerungsmittel (21) aufzunehmen, wobei der Keil stapelbar ist, damit man zwei oder mehr Keile auf dem Verankerungselement aufreihen und montieren kann,
bei dem der stapelbare Keil zwischen dem Kopf des Knochenverankerungselements und den Knochenverankerungsmitteln positioniert ist, und der stapelbare Keil ausgelegt ist, um auf den Kopf des Knochenverankerungsmittels montiert zu werden,
bei dem das Verankerungselement eine Einkerbung (23) umfasst und der stapelbare Keil außerdem eine Lippe (11;111;211;311) komplementär zur Einkerbung (23) umfasst, damit eine Verbindung der Lippe mit der Einkerbung eine Montage des Keils auf dem Knochenverankerungselement erlaubt.

2. Anordnung gemäß Anspruch 1, **dadurch gekennzeichnet, dass** ein Spalt (116; 216) derart angeordnet ist, dass der stapelbare Keil einen nicht geschlossenen Ring bildet.

3. Anordnung gemäß Anspruch 2, **dadurch gekennzeichnet, dass** der stapelbare Keil elastisch deformierbar ist, um die Einführung der Knochenverankerungsmittel durch den Spalt zu erlauben.

4. Anordnung gemäß einem der Ansprüche 1 bis 3, **dadurch gekennzeichnet, dass** die Lippe des stapelbaren Keils unterbrochen ist.

5. Anordnung gemäß einem der Ansprüche 1 bis 4, **dadurch gekennzeichnet, dass** der stapelbare Keil eine Einkerbung (13;113;213;313) umfasst, die komplementär zur Lippe (11;111;211;311) ist.

6. Anordnung gemäß Anspruch 5, **dadurch gekennzeichnet, dass** der stapelbare Keil ausgelegt ist, um auf einen anderen stapelbaren Keil montiert zu werden, durch Eingriff, jeweils der Lippe in die Einkerbung.

7. Anordnung gemäß einem der Ansprüche 5 oder 6, **dadurch gekennzeichnet, dass** wenigstens zwei stapelbare Keile gemeinsam montiert sind.

8. Anordnung gemäß Anspruch 7, **gekennzeichnet durch** einen ersten stapelbaren Keil, der erste Lippen und Einkerbungen umfasst, und einen zweiten stapelbaren Keil, der zweite Lippen und Einkerbungen umfasst, wobei der erste stapelbare Keil auf das Verankerungselement montiert ist durch Eingriff der ersten Lippe in die Einkerbung des Verankerungselements, und die zweite stapelbare Kerbe auf den ersten stapelbaren Keil **durch** Eingriff der zweiten Lippe in die erste Einkerbung montiert ist.

9. Anordnung gemäß Anspruch 8, **dadurch gekennzeichnet, dass** der zweite stapelbare Keil zwischen dem ersten stapelbaren Keil und den Knochenverankerungsmitteln positioniert ist.

10. Anordnung gemäß Anspruch 1, **dadurch gekennzeichnet, dass** eine Vielzahl von stapelbaren Keilen zwischen dem Kopf und den Knochenverankerungsmitteln positioniert ist.

11. Anordnung gemäß einem der Ansprüche 1 bis 10, **dadurch gekennzeichnet, dass** der stapelbare Keil ausgelegt ist, auf das Verankerungselement montiert zu werden für einen Eingriff durch elastische Deformation der Lippe des stapelbaren Keils auf der Einkerbung des Verankerungselements.
